# EUROPEAN PATENT SPECIFICATION

(11) **EP 4 462 543 B1**
(45) Date of publication and mention of the grant of the patent: **21.01.2026**
(21) Application number: 24173763.4
(22) Date of filing: 02.05.2024
(51) Int. Cl.: H01M 10/0567, H01M 10/0525, C07F 5/02, H01M 10/0568, H01M 4/58, H01M 10/0569, H01M 4/04

(54) **AN ELECTROLYTE ADDITIVE AND A PREPARATION METHOD THEREFOR, AN ELECTROLYTE AND A LITHIUM ION BATTERY**
ELEKTROLYTZUSATZ UND HERSTELLUNGSVERFAHREN DAFÜR, ELEKTROLYT UND LITHIUM-IONEN-BATTERIE
ADDITIF D'ÉLECTROLYTE ET SON PROCÉDÉ DE PRÉPARATION, ÉLECTROLYTE ET BATTERIE AU LITHIUM-ION

(30) Priority: 05.05.2023 CN 202310498087
(43) Date of publication of application: 13.11.2024
(73) Proprietor: Jiangsu Zenergy Battery Technologies Group Co., Ltd., Suzhou City, Jiangsu 215500 (CN)
(72) Inventor: ZHUANG, Sidong, Changshu Suzhou City, Jiangsu 215500 (CN); HUA, Bingyang, Changshu Suzhou City, Jiangsu 215500 (CN); DONG, Zhentao, Changshu Suzhou City, Jiangsu 215500 (CN); HE, Xingxing, Changshu Suzhou City, Jiangsu 215500 (CN); HUANG, Zeyu, Changshu Suzhou City, Jiangsu 215500 (CN)
(74) Representative: Novagraaf Group

(56) References cited:
- CN-B- 113 258 140
- QIAN WANG ET AL: "Dendrite-Free Lithium Deposition via a Superfilling Mechanism for High-Performance Li-Metal Batteries", ADVANCED MATERIALS, VCH PUBLISHERS, DE, vol. 31, no. 41, 29 August 2019 (2019-08-29), pages n/a, XP071818469, ISSN: 0935-9648, DOI: 10.1002/ADMA.201903248

## Description

### Technical Field

The present disclosure relates to the technical field of batteries, and in particular, to an electrolyte additive and a preparation method therefor, an electrolyte and a lithium ion battery.

### Background

An electrolyte, as an important constituent part of a lithium ion battery, directly affects the overall performance of the battery, such as cycle life, rate, cell internal resistance, capacity, and safety. A liquid electrolyte is generally composed of components such as a lithium salt, an organic solvent and an additive. The existing electrolyte has defects, resulting in that lithium dendrites easily precipitate on an electrode sheet, and the battery has poor cycle performance.

(XP071818469, ISSN: 0935-9648, DOI: 10.1002/ADMA.201903248) discloses Thiourea (THU) as electrolyte additive for lithium batteries, which enables dentrite free Lithium depositioh. Li cell can achieve stable cycling without any dendrite formation over 1000 cycles and 400 cycles at ultrahigh current densities of 5 mA cm⁻² and 10 mA cm⁻², respectively, manifesting one of the best results in Li metal anodes. In addition, the full-cell cycling performance with LiFePO₄ cathode also showed a very high capacity retention of 88% after 800 cycles at 1.0C and 90% after 650 cycles at 5.0 C.

(CN113258140 A) provides a lithium secondary battery electrolyte, and a preparation method and application thereof, and belongs to the technical field of batteries. The lithium secondary battery electrolyte comprises a conventional commercial ester electrolyte additive and a cosolvent, and the additive is nitrate and can participate in formation of an interface layer so as to improve the overall performance of the battery; and the cosolvent comprises a boron trifluoride-based complex, so that nitrate can be dissolved in an ester solvent, and the application range of the nitrate additive is widened. The lithium secondary battery electrolyte shows high reversible specific capacity and excellent cycling stability when being matched with a metal lithium battery assembled by a conventional NCM ternary material and a silicon-based negative electrode lithium ion battery, and is a practical electrolyte suitable for a novel high-specific-energy lithium secondary battery system.

In view of this, the present disclosure is specially proposed.

### Summary

An object of some embodiments of the present disclosure is to provide an electrolyte additive and a preparation method therefor, an electrolyte and a lithium ion battery, in which an electrolyte added with the electrolyte additive can effectively inhibit precipitation of lithium dendrites, and improve the battery cycle performance.

Some embodiments of the present disclosure are implemented as follows:
According to a first aspect, some embodiments of the present disclosure provide a preparation method for an electrolyte additive, the electrolyte additive being used for being added to an electrolyte of a lithium ion battery, and the preparation method for an electrolyte additive comprising:
Dissolving a picric acid in an organic solvent to obtain an additive solution, the organic solvent comprising at least one element of O, B, F and N; and
Performing crystallization from the additive solution, to precipitate the electrolyte additive; the organic solvent comprises at least one of an alcohol solvent, an ether solvent, and a ketone solvent.

In optional embodiments, the alcohol solvent comprises one or more of tetrafluoro p-methoxymethyl methanol, β-sitosterol, 2-bromo-9-methyl-9H-fluoren-9-ol, (1S,4R)-1-methyl-4-(1-methylvinyl)-2-cyclohexen-1-ol, 4-trifluoromethylbenzylboronic acid pinacol ester, tetramethylpiperidinol, triisopropanolamine and 2-(5-methylfuran-2-yl)ethanol;
The ether solvent comprises one or more of diethyl ether, ethylene glycol monomethyl ether, ethylene glycol monoethyl ether, ethylene glycol monobutyl ether, boron trifluoride diethyl ether, tetrafluoroborate diethyl ether, tetrafluoromethyl ether and tetrahydrofuran; and
The ketone solvent comprises one or more of 2-phenylchromone, flavonol, 2-hydroxychalcone, N-ethyl-2-pyrrolidone, isophorone and 2,4-pentanedione.

In optional embodiments, the step of dissolving the picric acid in the organic solvent comprises:
Adding the picric acid to the organic solvent and stirring same; and
Performing vacuumizing in a vacuum oven for performing a reaction.

In optional embodiments, the stirring temperature is 20-30 °C and the duration is 6-8 h.

In optional embodiments, the temperature in the vacuum oven is 60-80 °C.

In optional embodiments, the step of performing crystallization from the additive solution, to precipitate the electrolyte additive, comprises:
Performing crystallization by at least one method of rotary evaporator drying, oven drying, vacuum drying under reduced pressure, slow drying in an oil bath pan, and vacuum drying in a cryogenic vessel, to precipitate the electrolyte additive.

In optional embodiments, the step of performing crystallization from the additive solution, to precipitate the electrolyte additive, comprises:
Removing a solvent from the additive solution in an oven at 90-105 °C;
And/or, after vacuumizing, removing a solvent from the additive solution at 60-70 °C;
And/or, after increasing the temperature to 100 °C or higher in a rotary evaporator, stopping temperature increase, wrapping an oil bath pan with an aluminum foil, reducing the temperature to reach the room temperature after 6-8 h, then maintaining the temperature for 24-48 h, so that crystallization is performed in the additive solution to precipitate the electrolyte additive.

And/or, vacuumizing at -5 to -20 °C, to remove a solvent from the additive solution.

According to a second aspect, some embodiments of the present disclosure provide an electrolyte additive, which is prepared by the preparation method for an electrolyte additive according to any one of the embodiments above.

According to a third aspect, some embodiments of the present disclosure provide an electrolyte, the preparation raw material thereof comprising the electrolyte additive of the embodiments above.

According to a fourth aspect, some embodiments of the present disclosure provide a lithium ion battery, comprising the electrolyte of the embodiments above.

Some embodiments of the present disclosure have the following beneficial effects:

The preparation method for an electrolyte additive provided in some embodiments of the present disclosure comprises: dissolving a picric acid in an organic solvent to obtain an additive solution, the organic solvent comprising at least one element of B, F and N; and performing crystallization from the additive solution, to precipitate an electrolyte additive. As the organic solvent contains B, F and N and can be grafted into the structure of the picric acid, the electrolyte additive finally crystallized and precipitated is equivalent to substance after modification of the picric acid; the electrolyte additive contains a B element, an F element or an N element, and after being used in an electrolyte, the electrolyte additive can be polymerized to form BₓO_{y} (x=n, y=n+1) when a battery is charged and discharged, and at the same time, it is beneficial for an SEI film to generate of LiF, Li₃N, etc. of higher contents; these substances can be preferentially adsorbed on the tips of lithium dendrites, which helps to make a polymer layer and the SEI film more rigid and stable, and helps to suppress the growth of the lithium dendrites. The electrolyte added with the electrolyte additive can undergo an electropolymerization reaction during charging and discharging of the battery, thereby reducing the loss of the electrolyte and lithium metal, reducing the resistance of a lithium ion battery, and further improving the cycle performance of the lithium ion battery.

The lithium ion battery provided in some embodiments of the present disclosure comprises the electrolyte added with the electrolyte additive, and therefore has a lower resistance and better cycle performance.

### Brief Description of the Drawings

In order to illustrate the technical solutions of the embodiments of the present disclosure more clearly, hereinafter, accompanying drawings requiring to be used in the embodiments will be briefly introduced. It should be understood that the following accompanying drawings only illustrate certain embodiments of the present disclosure, and therefore shall not be considered as limiting the scope. For a person of ordinary skill in the art, other related accompanying drawings may also be obtained according to these accompany drawings without any inventive effort.
Fig. 1 is an SEM image of the surface of an electrolyte-lithium anode deposition layer of Comparative Embodiment 1; and
Fig. 2 is an SEM image of the surface of an electrolyte-lithium anode deposition layer according to Embodiment 5 of the present disclosure.

### Detailed Description of the Embodiments

To make the object, technical solutions and advantages of the embodiments of the present disclosure clearer, hereinafter, the technical solutions in the embodiments of the present disclosure will be clearly and completely described. If no specific conditions are noted in the embodiments, the experiments are performed according to conventional conditions or conditions suggested by a manufacturer. If manufacturers for reagents or instruments used are not noted, the reagents or instruments can be conventional products that can be commercially-available.

The features and performance in some embodiments of the present disclosure will be further described in detail below in combination with embodiments.

The preparation method for an electrolyte additive provided in embodiments of the present disclosure comprises:

Step S100, dissolving a picric acid in an organic solvent to obtain an additive solution, the organic solvent comprising at least one element of O, B, F and N.

In embodiments of the present disclosure, the organic solvent comprises at least one of an alcohol solvent, an ether solvent, and a ketone solvent.

Optionally, the alcohol solvent may be one or more of tetrafluoro p-methoxymethyl methanol, β-sitosterol, 2-bromo-9-methyl-9H-fluoren-9-ol, (1S,4R)-1-methyl-4-(1-methylvinyl)-2-cyclohexen-1-ol, 4-trifluoromethylbenzylboronic acid pinacol ester, tetramethylpiperidinol, triisopropanolamine and 2-(5-methylfuran-2-yl)ethanol;

The ether solvent may be one or more of diethyl ether, ethylene glycol monomethyl ether, ethylene glycol monoethyl ether, ethylene glycol monobutyl ether, boron trifluoride diethyl ether, tetrafluoroborate diethyl ether, tetrafluoromethyl ether and tetrahydrofuran; and

The ketone solvent may be one or more of 2-phenylchromone, flavonol, 2-hydroxychalcone, N-ethyl-2-pyrrolidone, isophorone and 2,4-pentanedione, etc.

The picric acid additive molecule comprises a C atom and an H atom, the C atom and the H atom are dissolved in an ether-based electrolyte, and may undergo redox, spontaneous reaction, catalytic induction, and similar compatibility, etc. The alcohol, ether and ketone solvents contain fluorine, oxygen, nitrogen, boron and metal ions, or are a mixture thereof; these components can be grafted in the structure of the picric acid, and finally can improve the protection of the electrolyte additive against an anode. Specifically, these components can make an electropolymerization layer more stable and uniform, or participate in an SEI film formation process, thereby generating higher contents of LiF, Li₂O, Li₃N, etc.

Further, the step of dissolving the picric acid in the organic solvent may specifically comprise:
Adding the picric acid to the organic solvent and stirring same; and performing vacuumizing in a vacuum oven for performing a reaction.

Optionally, the stirring temperature is 20-30 °C and the duration is 6-8 h. Optionally, the temperature in the vacuum oven is 60-80 °C. By a suitable stirring temperature, stirring duration and reaction temperature, the solubility and functionality of the picric acid in the organic solvent can be improved, which can assist some functional groups and chemical bonds to react.

The obtained additive solution is equivalent to a modified picric acid solution, as the additive solution contains a B element, an F element, metal ions or other various functional groups, the substance will undergo electropolymerization during charging and discharging of a battery, and BₓO_{y} (x=n, y=n+1) will be generated in the polymerization layer; the F element is bound with lithium ions in the electrolyte to generate LiF, and and metal ions can be preferentially adsorbed on the tips of lithium dendrites, which helps to make a polymer layer and the SEI film more rigid and stable, and helps to suppress the growth of the lithium dendrites.

Step S200: performing crystallization from the additive solution, to precipitate the electrolyte additive.

In embodiments of the present disclosure, the step S200 may comprise: performing crystallization by at least one method of rotary evaporator drying, oven drying, vacuum drying under reduced pressure, slow drying in an oil bath pan, and vacuum drying in a cryogenic vessel, to precipitate the electrolyte additive. Optionally, the crystallinity is controlled at 50%-65%.

For example, the electrolyte additive may be crystallized and precipitated in the following several manners:
1) Removing a solvent from the additive solution in an oven at 90-105 °C, wherein the heating rate can be optionally 5 °C per minute.
2) After vacuumizing, removing a solvent from the additive solution at 60-70 °C, wherein the heating rate can be optionally 5 °C per minute.
3) After increasing the temperature to 100 °C or higher in a rotary evaporator, stopping temperature increase, wrapping an oil bath pan with an aluminum foil, reducing the temperature to reach the room temperature after 6-8 h, then maintaining the temperature for 24-48 h, so that crystallization is performed in the additive solution to precipitate the electrolyte additive.
4) Vacuumizing at -5 to -20 °C, to remove a solvent from the additive solution; wherein an appropriate amount of liquid nitrogen and dry ice can be added to ensure uniform formation of crystals in the solution, and then the solvent is slowly removed by suction by a small amount of vacuum for 3-5 times and left for 24-48 h.

Hereinafter, in combination with various embodiments, the effect after the electrolyte additive prepared by using the preparation method for an electrolyte additive provided in some embodiments of the present disclosure is applied to a lithium ion battery will be described in detail.

### Embodiment 1

20 mg of picric acid was dissolved in 40 mL of 2-(5-methylfuran-2-yl)ethanol by stirring, and after drying by using a rotary evaporator, an electrolyte additive (about 50% of crystallinity) was obtained.

0.5 wt% of the electrolyte additive was used in an electrolyte base solution (not added with the electrolyte additive). A lithium salt in the electrolyte base solution is lithium bis(trifluoromethanesulfonyl)imide, the concentration of the lithium salt was 1.0 mol⁻¹, the solvent is 1,3-dioxolane and ethylene glycol dimethyl ether, and contained 0.5 wt% of lithium nitrate.

### Embodiment 2

10 mg of picric acid was dissolved in 20 mL of boron trifluoride diethyl ether by stirring, and the mixture was dried in an oven to raise the temperature from 25 °C to 90 °C at 5 °C per minute, and the temperature was maintained for 6 h to obtain an electrolyte additive in a powder form.

1 wt% of the electrolyte additive was used in an electrolyte base solution. The electrolyte base solution was the same as the electrolyte base solution in Embodiment 1.

### Embodiment 3

10 mg of picric acid was dissolved in 20 mL of N-ethyl-2-pyrrolidone by stirring, the mixture was vacuum-dried and vacuumized under reduced pressure, then the temperature was increased from an initial temperature of 25 °C to 60-70 °C at 5 °C per minute, and the temperature was maintained for 6 h to obtain an electrolyte additive in a powder form.

1.2 wt% of the electrolyte additive was used in an electrolyte base solution. The electrolyte base solution was the same as the electrolyte base solution in Embodiment 1.

### Embodiment 4

10 mg of picric acid was dissolved in 20 mL of 2-bromo-9-methyl-9H-fluoren-9-ol by stirring, the mixture was slowly dried by using an oil bath pan, and then transferred to a rotary evaporator for further drying, and the temperature was increased from an initial temperature of 25 °C to 100 °C at 5 °C per minute, and then the heating was stopped; the whole oil bath pan was wrapped with an aluminum foil so that the temperature thereof was slowly reduced, the temperature thereof was reduced to room temperature after 6-8 h, and the temperature was maintained for 24-48 h, and then an electrolyte additive (with a crystallinity of 55-60%) was crystallized and precipitated.

1.5 wt% of the electrolyte additive was used in an electrolyte base solution. The electrolyte base solution was the same as the electrolyte base solution in Embodiment 1.

### Embodiment 5

10 mg of picric acid was dissolved in 30 mL of a 4-trifluoromethylbenzylboronic acid pinacol ester solution by stirring, the mixture was vacuum-dried in a cryogenic vessel, and maintained at a low temperature of -10 °C. In this method, it is necessary to add an appropriate amount of liquid nitrogen and dry ice, to ensure uniform formation of crystals in the solution (about 65% of crystallinity). Then, the solvent was slowly removed by suction by a small amount of vacuum for 3-5 times and left for 24-48 h, to obtain the electrolyte additive.

2 wt% of the electrolyte additive was used in an electrolyte base solution. The electrolyte base solution was the same as the electrolyte base solution in Embodiment 1.

### Comparative Embodiment 1

No electrolyte additive was added to an electrolyte, that is, the electrolyte is the same as the electrolyte base solution in Embodiments 1-5.

### Comparative Embodiment 2

The difference from the embodiment 1 lied in that, picric acid was not pre-dissolved in an organic solvent containing at least one element of B, F and N and was not modified, but 0.5 wt% of picric acid was directly added to an electrolyte.

The electrolytes of the embodiments and comparative embodiments were applied to a battery, and the performance thereof was tested. The specific manner is as follows.

### 1. Preparation of a cathode plate:

(1) lithium iron phosphate, a conductive agent and a binder were mixed at a mass ratio of 8:1:1 and stirred, then an NMP solvent was added for uniform mixing to prepare a cathode slurry, the cathode slurry was uniformly coated at a certain ratio onto an aluminum foil coated with a conductive carbon layer, and vacuum drying was performed at 80-120 °C to obtain a cathode material coated with an active material layer. The binder is PVDF; and the conductive agent is one or more of SP, acetylene black and graphite.
(2) The cathode material in step 1 was die-cut to obtain a cathode plate.

2. An anode plate adopted a lithium metal sheet.

3. Battery preparation: a symmetrical battery was prepared by using two lithium metal sheets as counter electrodes; and a cathode plate, a separator and an anode plate were assembled together to form a battery, wherein the separator needs to completely wrap the cathode plate and the anode plate. An electrolyte was injected into the battery. Finally, a lithium iron phosphate battery was prepared.

### 4. Battery impedance test:

A battery impedance test was performed on batteries after cycling under a condition of 100 Hz.

### 5. Cycle test:

At 25±2 °C, a battery was charged to 3.65 V under a 1C constant current and a constant voltage, with a cut-off current being 0.05C; and the battery stood still for 60 min, then was discharged to 2.5V at 1C, the process continued until the capacity decayed to 80% of the initial capacity, and the number of cycles was recorded.

Table 1 was the parameters and test results of various embodiments and comparative embodiments.

**Table 1:**

| | Content of electrolyte additive (wt%) | Thickness of lithium anode deposition layer (µm) | Impedance value of battery (mΩ) | Cycle life (number of cycles) |
|---|---|---|---|---|
| Embodiment 1 | 0.5 | 17.56 | 0.367 | 3724 |
| Embodiment 2 | 1.0 | 35.32 | 0.703 | 3158 |
| Embodiment 3 | 1.2 | 37.48 | 0.741 | 3112 |
| Embodiment 4 | 1.5 | 15.53 | 0.313 | 3843 |
| Embodiment 5 | 2.0 | 14.84 | 0.281 | 3937 |
| Comparative Embodiment 1 | 0 | 43.37 | 0.935 | 2551 |
| Comparative Embodiment 2 | 0.5 (picric acid) | 38.62 | 0.786 | 2845 |

It could be determined from Table 1 that, after the electrolytes of Embodiments 1-5 in the present disclosure were applied to a battery, the deposition thickness of the lithium anode was thinner than that of the comparative embodiments; and the impedance value of the battery was lower than that of the comparative embodiments, and had better conductivity. Furthermore, compared with the comparative embodiments, the number of cycles was obviously improved, and therefore using the electrolyte additive provided in embodiments of the present disclosure could improve the cycle performance of a lithium ion battery.

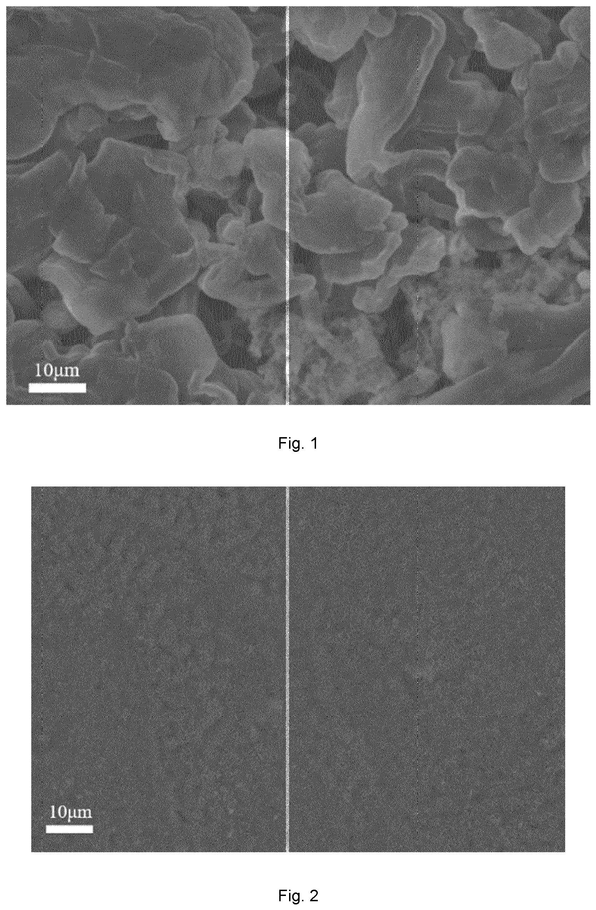

Fig. 1 was an SEM image of the surface of an electrolyte-lithium anode deposition layer of Comparative Embodiment 1. It could be determined from Fig. 1 that the irregular growth of lithium dendrites caused the deposition layer to be relatively loose and porous, which provided a place for poor chemical reactions between the electrolyte and the lithium metal, thereby causing excessive loss of the electrolyte and the lithium metal.

Fig. 2 was an SEM image of the surface of an electrolyte-lithium anode deposition layer according to Embodiment 5 of the present disclosure. It could be determined from Fig. 2 that, after the electrolyte additives of the embodiments of the present disclosure were used, the surface of the lithium anode after electropolymerization was relatively dense, thereby reducing side reactions between the electrolyte and the lithium metal.

Preparation raw materials of the electrolyte provided in embodiments of the present disclosure comprises the electrolyte additive prepared by the preparation method. In addition to having an electropolymerization function, the electrolyte additive provided in embodiments of the present disclosure could also participate in an electropolymerization reaction on the surface of an anode to generate a polymerization layer with stronger performance, thereby reducing adverse reactions between the electrolyte and lithium ions, reducing the thickness of a deposition layer on the surface of the anode, and reducing the internal impedance of the battery. Some functional groups and chemical bonds contained in the electrolyte additive could participate in the reaction of an anode SEI film, so that there were more inorganic substances such as Li₂O, LiF and Li₃N in the SEI film layer, which rendered the anode SEI film to be more robust, and could well inhibit the growth of lithium dendrites; moreover, the chemical bonds and functional groups carried a certain electrostatic force field, so that ion channels could be formed, facilitating uniform transmission of lithium ions, and being beneficial to improving the cycle performance and safety performance of the lithium battery. Furthermore, the electrolyte additive provided in the embodiments of the present disclosure has a simple preparation process and was easy to implement industrial production.

In addition, embodiments of the present disclosure further provide a lithium ion battery, which comprises the electrolyte provided by the embodiments of the present disclosure.

## Claims

1. A preparation method for an electrolyte additive, the electrolyte additive being used for being added to an electrolyte of a lithium ion battery, wherein the preparation method for an electrolyte additive comprises:
dissolving a picric acid in an organic solvent to obtain an additive solution, the organic solvent comprising at least one element of O, B, F and N; and
performing crystallization from the additive solution, to precipitate the electrolyte additive;
the organic solvent comprises at least one of an alcohol solvent, an ether solvent, and a ketone solvent.

2. The preparation method for an electrolyte additive according to claim 1, wherein the alcohol solvent comprises one or more of tetrafluoro p-methoxymethyl methanol, β-sitosterol, 2-bromo-9-methyl-9H-fluoren-9-ol, (1S,4R)-1-methyl-4-(1-methylvinyl)-2-cyclohexen-1-ol, 4-trifluoromethylbenzylboronic acid pinacol ester, tetramethylpiperidinol, triisopropanolamine and 2-(5-methylfuran-2-yl)ethanol;
the ether solution comprises one or more of diethyl ether, ethylene glycol monomethyl ether, ethylene glycol monoethyl ether, ethylene glycol monobutyl ether, boron trifluoride diethyl ether, tetrafluoroborate diethyl ether, tetrafluoromethyl ether and tetrahydrofuran; and
the ketone solution comprises one or more of 2-phenylchromone, flavonol, 2-hydroxychalcone, N-ethyl-2-pyrrolidone, isophorone and 2,4-pentanedione.

3. The preparation method for an electrolyte additive according to claim 1, wherein the step of dissolving the picric acid in the organic solvent comprises:
adding the picric acid to the organic solvent and stirring same; and
performing vacuumizing in a vacuum oven for performing a reaction.

4. The preparation method for an electrolyte additive according to claim 3, wherein the stirring temperature is 20-30 °C and the duration is 6-8 h.

5. The preparation method for an electrolyte additive according to claim 3, wherein the temperature in the vacuum oven is 60-80 °C.

6. The preparation method for an electrolyte additive according to claim 1, wherein the step of performing crystallization from the additive solution, to precipitate the electrolyte additive, comprises:
performing crystallization by at least one method of rotary evaporator drying, oven drying, vacuum drying under reduced pressure, slow drying in an oil bath pan, and vacuum drying in a cryogenic vessel, to precipitate the electrolyte additive.

7. The preparation method for an electrolyte additive according to claim 6, wherein the step of performing crystallization from the additive solution, to precipitate the electrolyte additive, comprises:
removing a solvent from the additive solution in an oven at 90-105 °C;
and/or, after vacuumizing, removing a solvent from the additive solution at 60-70 °C;
and/or, after increasing the temperature to 100 °C or higher in a rotary evaporator, stopping temperature increase, wrapping an oil bath pan with an aluminum foil, reducing the temperature to reach the room temperature after 6-8 h, then maintaining the temperature for 24-48 h, so that crystallization is performed in the additive solution to precipitate the electrolyte additive;
and/or, vacuumizing at -5 °C to -20 °C to remove the solvent from the additive solution.

8. An electrolyte additive, wherein the electrolyte additive is prepared by using the preparation method for an electrolyte additive according to any one of claims 1-7.

9. An electrolyte, wherein preparation raw materials of the electrolyte comprise the electrolyte additive according to claim 8.

10. A lithium ion battery, wherein the lithium ion battery comprises the electrolyte according to claim 9.

11. The preparation method for an electrolyte additive according to claim 6, the solvent is slowly removed by suction by a small amount of vacuum for 3-5 times and left for 24-48 h.

12. The preparation method for an electrolyte additive according to claim 6, wherein, the crystallinity of the crystallization is controlled at 50%-65%.

## Patentansprüche

1. Herstellungsverfahren für einen Elektrolytzusatzstoff, wobei der Elektrolytzusatzstoff zum Zugeben zu einem Elektrolyten einer Lithiumionenbatterie verwendet wird, wobei das Herstellungsverfahren für einen Elektrolytzusatzstoff umfasst:
Auflösen einer Pikrinsäure in einem organischen Lösungsmittel, um eine Zusatzstofflösung zu erhalten, das organische Lösungsmittel umfassend mindestens ein Element aus O, B, F und N; und
Durchführen einer Kristallisation aus der Zusatzstofflösung, um den Elektrolytzusatzstoff auszufällen;
das organische Lösungsmittel mindestens eines von einem Alkohollösungsmittel, einem Etherlösungsmittel und einem Ketonlösungsmittel umfasst.

2. Herstellungsverfahren für einen Elektrolytzusatzstoff nach Anspruch 1, wobei das Alkohollösungsmittel eines oder mehrere von Tetrafluor-p-methoxymethylmethanol, β-Sitosterol, 2-Brom-9-methyl-9H-fluoren-9-ol, (1S,4R)-1-Methyl-4-(1-methylvinyl)-2-cyclohexen-1-ol, 4-Trifluormethylbenzylboronsäurepinakolester, Tetramethylpiperidinol, Triisopropanolamin und 2-(5-Methylfuran-2-yl)ethanol umfasst;
die Etherlösung einen oder mehrere von Diethylether, Ethylenglykolmonomethylether, Ethylenglykolmonoethylether, Ethylenglykolmonobutylether, Bortrifluoriddiethylether, Tetrafluoroboratdiethylether, Tetrafluoromethylether und Tetrahydrofuran umfasst; und
die Ketonlösung eines oder mehrere von 2-Phenylchromon, Flavonol, 2-Hydroxychalcon, N-Ethyl-2-pyrrolidon, Isophoron und 2,4-Pentandion umfasst.

3. Herstellungsverfahren für einen Elektrolytzusatzstoff nach Anspruch 1, wobei der Schritt des Auflösens der Pikrinsäure in dem organischen Lösungsmittel umfasst:
Zugeben der Pikrinsäure zu dem organischen Lösungsmittel und Rühren desselben; und
Durchführen einer Vakuumierung in einem Vakuumofen zum Durchführen einer Reaktion.

4. Herstellungsverfahren für einen Elektrolytzusatzstoff nach Anspruch 3, wobei die Rührtemperatur 20-30 °C beträgt und die Dauer 6-8 h beträgt.

5. Herstellungsverfahren für einen Elektrolytzusatzstoff nach Anspruch 3, wobei die Temperatur in dem Vakuumofen 60-80 °C beträgt.

6. Herstellungsverfahren für einen Elektrolytzusatzstoff nach Anspruch 1, wobei der Schritt des Durchführens der Kristallisation aus der Zusatzstofflösung, um den Elektrolytzusatzstoff auszufällen, umfasst:
Durchführen der Kristallisation durch mindestens ein Verfahren aus Rotationsverdampfertrocknung, Ofentrocknung, Vakuumtrocknung unter vermindertem Druck, langsamer Trocknung in einer Ölbadwanne und Vakuumtrocknung in einem kryogenen Gefäß, um den Elektrolytzusatzstoff auszufällen.

7. Herstellungsverfahren für einen Elektrolytzusatzstoff nach Anspruch 6, wobei der Schritt des Durchführens einer Kristallisation aus der Zusatzstofflösung, um den Elektrolytzusatzstoff auszufällen, umfasst:
Entfernen eines Lösungsmittels aus der Zusatzstofflösung in einem Ofen bei 90-105 °C;
und/oder nach der Vakuumierung, Entfernen eines Lösungsmittels aus der Zusatzstofflösung bei 60-70 °C;
und/oder, nach einem Erhöhen der Temperatur auf 100 °C oder höher in einem Rotationsverdampfer, Stoppen der Temperaturerhöhung, Umhüllen einer Ölbadwanne mit einer Aluminiumfolie, Reduzieren der Temperatur, um die Raumtemperatur nach 6-8 h zu reduzieren, dann Aufrechterhalten der Temperatur für 24-48 h, so dass die Kristallisation in der Zusatzstofflösung durchgeführt wird, um den Elektrolytzusatzstoff auszufällen;
und/oder Vakuumierung bei -5 °C bis -20 °C, um das Lösungsmittel aus der Zusatzstofflösung zu entfernen.

8. Elektrolytzusatzstoff, wobei der Elektrolytzusatzstoff durch Verwenden des Herstellungsverfahrens für einen Elektrolytzusatzstoff nach einem der Ansprüche 1-7 hergestellt wird.

9. Elektrolyt, wobei Herstellungsrohstoffe des Elektrolyts den Elektrolytzusatzstoff nach Anspruch 8 umfassen.

10. Lithiumionenbatterie, wobei die Lithiumionenbatterie den Elektrolyten nach Anspruch 9 umfasst.

11. Herstellungsverfahren für einen Elektrolytzusatzstoff nach Anspruch 6, wobei das Lösungsmittel durch Absaugen durch eine geringe Menge von Vakuum 3-5 Mal langsam entfernt und 24-48 h stehen gelassen wird.

12. Herstellungsverfahren für einen Elektrolytzusatzstoff nach Anspruch 6, wobei die Kristallinität der Kristallisation bei 50 %-65 % kontrolliert wird.

## Revendications

1. Procédé de préparation d'un additif d'électrolyte, l'additif d'électrolyte étant utilisé pour être ajouté à un électrolyte d'une batterie lithium-ion, dans lequel le procédé de préparation d'un additif d'électrolyte comprend :
la dissolution d'un acide picrique dans un solvant organique pour obtenir une solution d'additif, le solvant organique comprenant au moins un élément parmi O, B, F et N ; et
la réalisation d'une cristallisation à partir de la solution d'additif, afin de précipiter l'additif d'électrolyte ;
le solvant organique comprend au moins l'un parmi un solvant d'alcool, solvant d'éther et solvant de cétone.

2. Procédé de préparation d'un additif d'électrolyte selon la revendication 1, dans lequel le solvant alcoolique comprend un ou plusieurs parmi tétrafluoro p-méthoxyméthyl méthanol, β-sitostérol, 2-bromo-9-méthyl-9H-fluoren-9-ol, (1S,4R)-1-méthyl-4-(1-méthylvinyl)-2-cyclohexène-1-ol, ester de pinacol de l'acide 4-trifluorométhylbenzylboronique, tétraméthylpipéridinol, triisopropanolamine et 2-(5-méthylfuran-2-yl)éthanol ;
la solution d'éther comprend un ou plusieurs parmi éther diéthylique, éther monométhylique d'éthylène glycol, éther monoéthylique d'éthylène glycol, éther monobutylique d'éthylène glycol, éther diéthylique de trifluorure de bore, éther diéthylique de tétrafluoroborate, éther tétrafluorométhylique et tétrahydrofuranne ; et
la solution de cétone comprend un ou plusieurs parmi 2-phénylchromone, flavonol, 2-hydroxychaicone, N-éthyl-2-pyrrolidone, isophorone et 2,4-pentanedione.

3. Procédé de préparation d'un additif d'électrolyte selon la revendication 1, dans lequel l'étape de dissolution de l'acide picrique dans le solvant organique comprend :
l'ajout de l'acide picrique au solvant organique et l'agitation ; et
la mise sous vide dans une étuve à vide pour effectuer une réaction.

4. Procédé de préparation d'un additif d'électrolyte selon la revendication 3, dans lequel la température d'agitation est de 20 à 30 °C et la durée de 6 à 8 h.

5. Procédé de préparation d'un additif d'électrolyte selon la revendication 3, dans lequel la température de l'étuve à vide est de 60 à 80 °C.

6. Procédé de préparation d'un additif d'électrolyte selon la revendication 1, dans lequel l'étape de cristallisation à partir de la solution d'additif, pour précipiter l'additif d'électrolyte, comprend :
la cristallisation par au moins un procédé de séchage à l'évaporateur rotatif, de séchage à l'étuve, de séchage sous vide sous pression réduite, de séchage lent dans un bain d'huile et de séchage sous vide dans un récipient cryogénique, afin de précipiter l'additif d'électrolyte.

7. Procédé de préparation d'un additif d'électrolyte selon la revendication 6, dans lequel l'étape de cristallisation à partir de la solution d'additif, pour précipiter l'additif d'électrolyte, comprend :
l'élimination d'un solvant de la solution d'additif dans une étuve à une température comprise entre 90 et 105 °C ;
et/ou, après mise sous vide, l'élimination d'un solvant de la solution d'additif à une température comprise entre 60 et 70 °C ;
et/ou, après avoir augmenté la température à 100 °C ou plus dans un évaporateur rotatif, l'arrêt de l'augmentation de la température, l'enveloppe d'un bac de bain d'huile avec une feuille d'aluminium, la réduction de la température pour atteindre la température ambiante après 6 à 8 h, puis le maintien de la température pendant 24 à 48 h, de sorte que la cristallisation est effectuée dans la solution d'additif pour précipiter l'additif d'électrolyte ;
et/ou, la mise sous vide à une température comprise entre -5 °C et -20 °C pour éliminer le solvant de la solution d'additif.

8. Additif d'électrolyte, dans lequel l'additif d'électrolyte est préparé à l'aide du procédé de préparation d'un additif d'électrolyte selon l'une quelconque des revendications 1 à 7.

9. Électrolyte, dans lequel les matières premières de préparation de l'électrolyte comprennent l'additif d'électrolyte selon la revendication 8.

10. Batterie au lithium-ion, dans laquelle la batterie au lithium-ion comprend l'électrolyte selon la revendication 9.

11. Procédé de préparation d'un additif d'électrolyte selon la revendication 6, le solvant est lentement éliminé par aspiration à l'aide d'une petite quantité de vide pendant 3 à 5 fois et laissé pendant 24 à 48 heures.

12. Procédé de préparation d'un additif d'électrolyte selon la revendication 6, dans lequel la cristallinité de la cristallisation est contrôlée à hauteur de 50 % à 65 %.
